# EUROPEAN PATENT SPECIFICATION

(11) **EP 0 327 928 B1**
(45) Date of publication and mention of the grant of the patent: **02.03.1994**
(21) Application number: 89101625.5
(22) Date of filing: 31.01.1989
(51) Int. Cl.: A61H 1/00

(54) **Bladder Evacuator**
Blasenevakuator
Evacuateur de la vessie

(30) Priority: 10.02.1988 US 154192
(43) Date of publication of application: 16.08.1989
(73) Proprietor: BOSTON SCIENTIFIC CORPORATION, Watertown, MA 02172-2407 (US); Roth, Robert A., Brookline, MA 02167 (US)
(72) Inventor: Roth, Robert A., MD, Brookline, MA 02167 (US); Clement, Thomas P., Bloomington Indiana 47401 (US)
(74) Representative: Bardehle, Heinz, Dipl.-Ing.

(56) References cited:
- EP-A- 0 202 384
- EP-A- 0 254 410
- FR-A- 2 592 584

## Description

The invention relates to a bladder evacuator according to the first part of claim 1.

E. g. , in the field of urological surgery, the need frequently arises to evacuate, by flushing, pieces of solid or semi-solid material from the urinary tract, e.g., the bladder.

Devices in the past have removed particles, such as stones, blood clots, pieces of tissue and the like by first introducing a sterile fluid such as saline solution into a body cavity and thereafter withdrawing the fluid to flush the particles from the body.
The mixture is collected in a chamber and must be strained or filtered
before the fluid is recirculated into the body cavity for collection of further particles.

One particular application is flushing free-floating prostatic tissue chips from the bladder following a transurethral resectioning (TUR) procedure.

A bladder evacuator according to the first part of claim 1 has become known from **EP -A2- 0 254 410** (BARD LTD.).

The technical **problem** of the invention regarding the prior art is to **avoid**
the delay between flushing strokes required in many prior art devices in which the tissue must be allowed time to settle from the withdrawn fluid
before it can be returned to the body, and also
the requirement for moving parts such as flap-type check valves and the like which may become clogged.

The **solution** to this problem is accomplished by
the **characterizing part** of **Claim 1**.

As a result of the orientation of the elongated barrel of the filter, i.e.,
with the conduit of the neck, the filter body, and the opening at the outer end of the filter disposed coaxially along the axis of the container,
tissue-carrying fluid drawn from the body flows along this unrestricted passageway into the volume of the container through the opening at the outer end of the filter.

The length of the filter extending into the volume of the container, and thus
the remote position of the open outer end of the filter relative to the region at the side of the container where the compressional forces are applied,
results in fluid displaced by the compressional forces being directed against the outer surface of the side wall of the filter, with the fluid flowing through the small openings.

Since little or none of the tissue-carrying fluid is forced to enter the outer open end of the filter, and
since the small openings prevent passage of tissue pieces from the volume of the container through the sidewalls of the filter,
the flushing fluid re-entering the body is relatively free of pieces of tissue.

Thus by repeated squeezing and release of the container, tissue is flushed for the body and delivered into the container with the fluid, the pieces of tissue are collected in the container, and the fluid, free of tissue pieces, may then be returned back into the body for flushing additional pieces of tissue to be returned to the container.

The **remaining Claims** go further in specifying the invention.

A plurality of ways of carrying out the invention is described in detail below with reference to **drawings,** namely:
- **Fig. 1**: is an exploded perspective view of
a bladder evacuator of the invention;
- **Figs. 2,3,4**: are side views, partially in section, of the bladder evacuator,

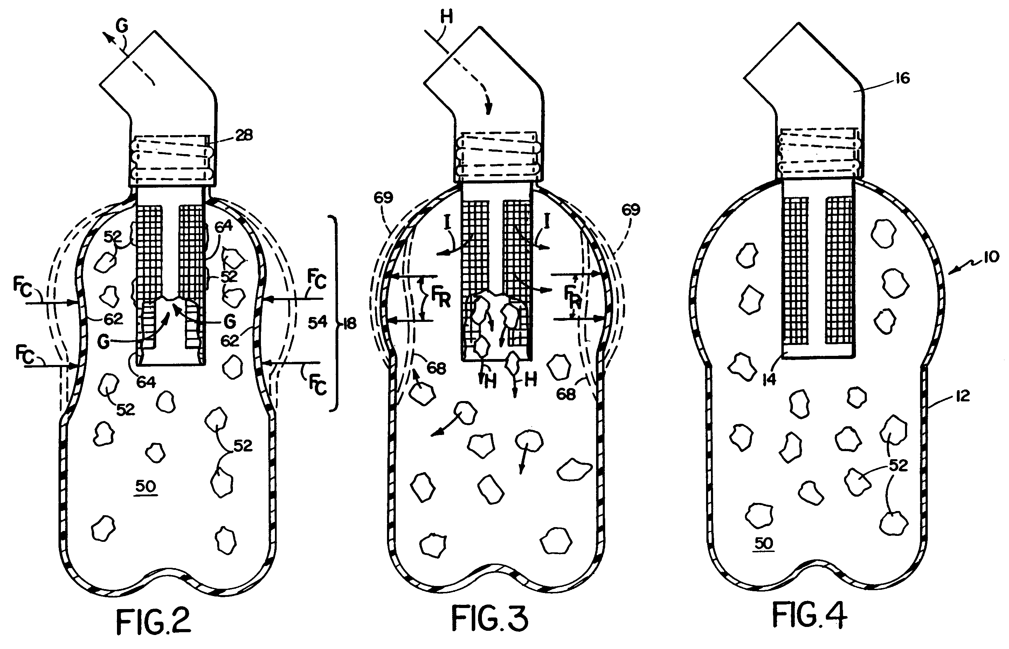

- **Fig. 2**: showing application of transverse compressional forces
for introduction of essentially tissue-particle-free fluid into a body,
- **Fig. 3**: showing release of compressional forces
to allow tissue-particle-bearing fluid to be withdrawn from the body,
- **Fig. 4**: showing the bladder evacuator briefly
between release and re-application of compressional forces; and
- **Fig. 5**: is a perspective view of
an alternate embodiment of the evacuator.

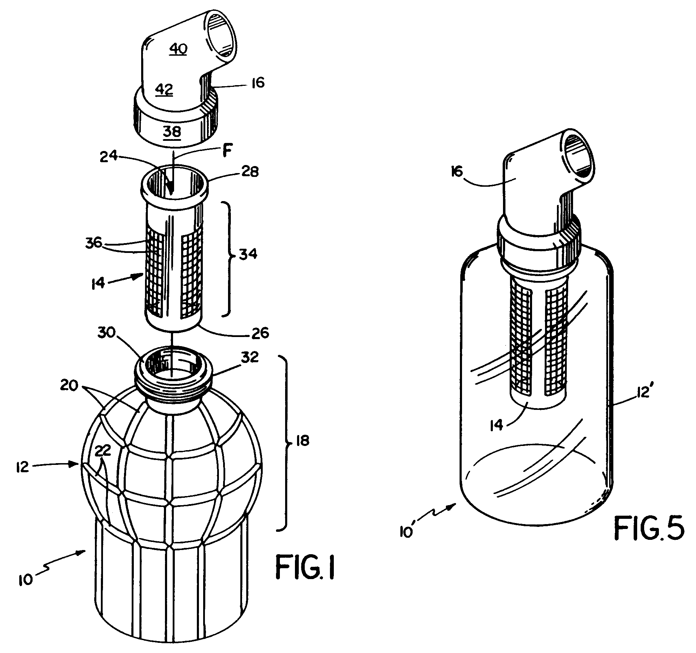

Referring to Fig. 1, a bladder evacuator 10 of the invention for quickly and efficiently removing particles from a body cavity, e.g., prostatic tissue chips after a transurethral resectioning (TUR) procedure, consists of a squeezable, flexible container 12, a filter element 14 extending within container 12, and a connector 16.

Container 12 is generally cylindrical and is sized to hold about 12 ounces of irrigation fluid. Preferably, the container is formed of resilient material, e.g., polyvinyl chloride (PVC), polyethylene or polypropylene, with a wall thickness of about 4 mm (0.156 inch), and is transparent to allow the user to monitor the fluid. At least the upper segment 18 of the container consists a squeezable, ribbed portion, preferably disposed generally coaxially with the longitudinal axis F of filter element 14. Vertical and horizontal ribs 20, 22 along the exterior surface of container 12 provide increased resiliency.

Filter element 14 consists of a molded plastic cylinder open at the top 24 and having a filter opening 26 at the bottom. A flange 28 about the perimeter of the top opening is sized to rest on the top lip 30 of the neck 32 of the container 12. Filter 14 includes a screen area 34 extending generally about its circumference and along its length, having pores 36 sized to restrict throughflow of tissue chips and other particles above a predetermined size, e.g., pores 36 are approximately 0.5 to 1.0 millimeter square.

Connector 16, e.g. also of plastic, has a threaded base 38 for threading onto neck 32 of container 12 and engages upon flange 28 to hold filter 14 in place. Connector 16 has upper and lower portions 40, 42 disposed at angle D (Fig. 2), e.g., about 45°, for connection to a working channel of an endoscope (not shown).

The container 12 (Fig. 1) of the bladder evacuator 10 is filled with an irrigation fluid, e.g., saline solution, and assembled with the filter element 14 and connector 16. An endoscope is advanced to the surgical site as the physican observes through the viewing channel of the endoscope. Connector 16 is connected to the working channel of the endoscope to provide a conduit for fluid from the container to the site.

Referring now to FIG. 2, the bladder evacuator 10 is shown during a flushing procedure, and contains fluid 50 and numerous chip 52 of tissue removed from the body. Compressional forces (arrows F_{c}) of at least a minimum magnitude, e.g., 1 - 2 Kp (2 to 4 lbs.), are applied to upper portion 18 of container 12 to displace the container walls 54 in region 18 from their initial dashed line position inwardly, toward axis F of filter element 14, to cause the irrigation fluid 50 to flow toward the filter and through the filter pores 36. The pores 36 are sized to restrict through-flow of tissue chips 52 above a predetermined size, and application of the compressional forces F_{c} upon portion 18 of the container causes substantially only the fluid within the upper portion 18 of the container, between the container wall 64 and the filter surface 62 to flow directly toward the filter surface; fluid in the lower portion 66 of the container is essentially undisturbed. The fluid (arrows G) passes through the filter pores (leaving tissue chips 52 behind) and through the top opening 24 of the filter, out the open neck of the container and thereafter from opening 26 of connector 16 into the working channel of the endoscope, and therethrough into the bladder of a patient.

Referring now to FIG. 3, after the walls 54 of the upper portion 18 of the container 12 are compressed to their innermost position adjacent the surface 64 of the filter element (indicated by dashed lines 68), the compressional forces (F_{c}, Fig. 2) are released, and the resilient forces of the container (arrows F_{R}) cause the container walls to move outwardly, toward their original positions (dashed lines 69). The bladder evacuator and conduit are a closed system, and movement of the walls acts to withdraw the flushing fluid from within the body, carrying with it tissue chips removed in surgery. The tissue carrying fluid enters the evacuator 10 via the connector 16 to flow along the axis F of the filter, and into the container 12 through the filter opening 26 of filter element 14. (The direction and force of flow (arrows H) into the container serves to sweep a substantial portion of the tissue chips 52 from within the filter element 14, even as a small portion (arrows I) of the fluid is drawn through the pores 36 of the surface 64 of the filter.

Referring now to FIG. 4, after release, (FIG. 3) and immediately before reapplication of compressional forces (FIG. 2), the container 12 is full of tissue-chip bearing fluid, typically in random movement. A short pause between cycles allows some settling of tissue chips 52, e.g., from within the volume of the filter element 14, but little, or no delay if desired, is necessary before the fluid is again forced through the pores 36 of filter 14, and the returning irrigation fluid is substantially free of tissue chips and particles above the predetermined size.

The irrigation procedure can thus be carried out quickly, with little delay required between flushing strokes, and with little backflow of tissue particles into the body.

Other embodiments are within the scope of the following claims. For example, bladder evacuator 10′ may have a container 12′ in the form of a right cylinder (FIG. 5).

## Claims

1. **Bladder evacuator** (10) (*Fig 1*) for
- alternately introducing fluid (50) into and withdrawing fluid from a
- bladder cavity
- through a conduit ,
*comprising*
- a **container** (12) for **fluid**
- defining
- a fluid-receiving volume and
- having
- a **neck opening** (32),
- **filter means**
- disposed within the volume of said container (12)
- for separation of tissue particles (52) above a predetermined size
- from fluid introduced from said volume through said neck opening (32), and
- **cap means** (16)
- for interconnecting said neck opening (32) and said conduit;
- an elongated **filter element** (14) of said **filter means**
- being disposed adjacent said neck opening (32) and
- extending within the volume of said container (12) and
- being disposed about the **axis** (F) of said neck opening (32) and
- defining
- two **axial openings** (24, 26),
- an **upper** one (24) to the neck opening (32) and
- a **lower** one (26) spaced therefrom,
- such that an axial passageway is defined between said two openings,
- said passageway functioning as a bypass,
- so that material flowing through said passageway
- does not pass through said filter element (14);
- said **bladder evacuator** (10) being adapted
- for **application** of **compressional forces** (F_{c}) (*Fig. 2*) upon said container (12)
- in directions **transverse** to said axis (F) of said filter element (14)
- for urging fluid (G) within the volume of said container (12)
- substantially free of tissue particles (52) in excess of said predetermined size,
- to pass through said neck opening (32) into said conduit toward said bladder cavity, and
- upon **release** of said **compressional forces** (F_{c}) (*Fig. 3*),
- said container (12) being adapted
- to expand
- to withdraw unfiltered tissue particle bearing fluid (H)into the volume of said container (12)
- via said neck opening (32),
*characterized* in that
- said **container** (12) is adapted
- to be compressed by said compressional forces (F_{c})
- at an **upper portion** (18) thereof, whereas
- the **length** of said filter element (14)
- extending into the volume of said container (12) is such that
- said **lower** axial **opening** (26) of said filter element (14) is
- **remotedly** positioned (i.e. in a lower location)
- relative to
- said **upper portion** (18) of said container (12)
- where said **compressional forces** (F_{c}) are applied,
- the arrangement resulting in
- said **fluid (G)** *(Fig. 2)*
- urged by said compressional forces (F_{c})
- being directed against the outer surface of the side wall of said filter element (14),
- with said fluid (G) flowing through its **filter pores** (36),
- whereas upon **release** of said compressional forces (F_{c}) *(Fig. 3)*
- said **unfiltered** tissue particles bearing fluid **(H)** flows
- generally **axially** within said **filter element** (14)
- through its **lower** axial opening (26),
- by passing its said **filter pores** (36).

2. Bladder evacuator of claim 1,
*characterized in* that
- said **container** (12) comprises
- a generally cylindrical body portion.

3. Bladder evacuator of claim 1,
*characterized in* that
- said **filter element** (14) is
- generally cylindrical.

4. Bladder evacuator of claim 1,
*characterized in* that
- **side walls** of said container (12) and of said filter element (14) are
- substantially parallel.

5. Bladder evacuator of claim 1,
*characterized in* that
- said **container** (12) is comprised of
- **resilient material**.

6. Bladder evacuator of claim 5,
*characterized in* that
- said **resilient material** is
- a **polymeric** material
- selected from the group of
- polyvinyl chloride (PVC), polyethylene and polypropylene.

7. Bladder evacuator of claim 1
*characterized in* that
- said **cap means** (16) comprises
- a **first portion** (40)
- extending from said **open neck** (32) and
- a **second portion** (42)
- more adjacent to said **conduit**,
- the **axes** of said first portion (40) and said second portion (42) defining
- an **acute angle** (D).

8. Bladder evacuator of claim 7,
*characterized in* that
- said **angle** (D) is of the order of
- about **45**°

9. Bladder evacuator of claim 1,
*characterized in* that
- said **filter element** (14) comprises
- a **mesh** (34)
- having
- said **filter pore openings** (36)
- of the order of about **1 mm** or less.

10. Bladder evacuator of claim 9,
*characterized in* that
- said **filter pore openings** (36) are
- about **0.5 to 1.0 mm**.

11. Bladder evacuator of claim 1,
*characterized in* that
- said **conduit** comprises
- a working channel of an endoscope.

## Patentansprüche

1. Blasenevakuator (10) (Fig.1) zum wechselweise Einführen von Fluid (50) in einem und Abziehen von Fluid aus einem Blasenhohlraum durch eine Leitung, der aufweist:
einen Behälter (12) für Fluid, der ein Fluid aufnehmendes Volumen definiert und eine Halsöffnung (32) besitzt,
eine Filtereirrichtung, die innerhalb des Volumens des Behälter (12) angeordnet ist zum Trennen von Gewebepartikeln (52) über einer vorbestimmten Größe von einem von dem Volumen durch die Halsöffnung (32) eingeführten Fluid und
eine Kappeneinrichtung (16) zum untereinander Verbinden von der Halsöffnung (32) und der Leitung;
wobei ein längliches Filterelement (14) der Filtereinrichtung benachbart zu der Halsöffnung (32) angeordnet ist und sich innerhalb des Volumens des Behälters (12) erstreckt und über der Achse (F) der Halsöffnung (32) angeordnet ist und zwei axiale Öffnungen (24, 26), eine obere (24) zu der Halsöffnung (32) und eine untere (26) davon beabstandet definiert,
so daß ein axialer Durchgang zwischen den zwei Öffnungen definiert wird, wobei der Durchgang als ein Bypass funktioniert, so daß Material, das durch den Durchgang fließt, nicht durch das Filterelement (14) hindurchgeht;
wobei der Blasenevakuator (10) angepaßt ist zum Anlegen von Kompressionskräften (F_{c}) (Fig.2) an den Behälter (12) in Richtungen quer zu der Achse (F) des Filterelements (14) zum Drängen von Fluid (G) innerhalb des Volumens des Behälters (12), das im wesentlichen frei von Gewebepartikeln (52) über der vorbestimmten Größe hinaus ist um durch die Halsöffnung (32) in die Leitung in Richtung auf den Blasenhohlraum zu gelangen,
und auf die Freigabe der Kompressionskräfte (F_{c}) hin (Fig.3) wird der Behälter (12) angepaßt zu expandieren, um ungefiltertes Fluid (H), das Gewebepartikel trägt, in das Volumen des Behälters (12) über die Halsöffnung (32) abzuziehen,
**dadurch gekennzeichnet, daß**
der Behälter (12) angepaßt ist, durch die Kompressionskräfte (F_{c}) an einem oberen Abschnitt (18) davon zusammengedrückt zu werden, wohingegen die Länge des Filterelements (14), die sich in das Volumen des Behälters (12) erstreckt' so ist, daß die untere axiale Öffnung (26) des Filterelements (14) entfernt (d.h. in einem unteren Ort) relativ zu dem oberen Abschnitt (18) des Behälters (12) angeordnet ist, wo die Kompressionskräfte (F_{c}) angelegt werden,
wobei die Anordnung dazu führt, daß das Fluid (G) (Fig.2), das durch die Kompressionskräfte (F_{c}) gedrängt wird, gegen die äußere Oberfläche der Seitenwand des Filterelementes (14) gerichtet ist,
wobei das Fluid (G) durch seine Filterporen (36) fließt, woraufhin auf die Freigabe der Kompressionskräfte (F_{c}) hin (Fig.3) das ungefilterte Fluid (H), das Gewebepartikel trägt, im allgemeinen axial innerhalb des Filterelementes (14) durch seine untere axiale Öffnung (26) durch Hindurchströmen durch seine Filterporen (36) strömt.

2. Blasenevakuator gemäß Anspruch 1, dadurch gekennzeichnet, daß der Behälter (12) im allgemeinen einen zylindrischen Körperabschnitt aufweist.

3. Blasenevakuator gemäß Anspruch 1, dadurch gekennzeichnet, daß das Filterelement (14) im allgemeinen zylindrisch ist.

4. Blasenevakuator gemaß Anspruch 1, dadurch gekennzeichnet, daß Seitenwände des Behälters (12) und des Filterelementes (14) im wesentlichen parallel sind.

5. Blasenevakuator gemaß Anspruch 1, dadurch gekennzeichnet, daß der Behälter (12) aus elastischem Material besteht.

6. Blasenevakuator gemaß Anspruch 5, dadurch gekennzeichnet, daß das elastische Material ein Polymermaterial ist, das aus der Gruppe von Polyvinlchlorid (PVC), Polyethylen und Polypropylen ausgewählt ist.

7. Blasenevakuator gemäß Anspruch 1, dadurch gekennzeichnet, daß die Kappeneinrichtung (16) einen ersten Abschnitt (40), der sich von dem offenen Hals (32) erstreckt, und einen zweiten Abschnitt (42) aufweist, der benachbarter zu der Leitung ist, wobei die Achsen des ersten Abschnittes (40) und des zweiten Abschnittes (42) einen spitzen Winkel (D) bilden.

8. Blasenevakuator gemäß Anspruch 7, dadurch gekennzeichnet, daß der Winkel (D) in der Größenordnung von etwa 45° ist.

9. Blasenevakuator gemäß Anspruch 1, dadurch gekennzeichnet, daß das Filterelement (14) ein Sieb (34) mit den Filterporenöffnungen (36) der Größenordnung von etwa 1 mm oder Kleiner aufweist.

10. Blasenevakuator gemäß Anspruch 9, dadurch gekennzeichnet, daß die Filterporenöffnungen (36) etwa 0,5-1,0 mm sind.

11. Blasenevakuator gemäß Anspruch 1, dadurch gekennzeichnet, daß die Leitung einen Arbeitskanal eines Endoskops aufweist.

## Revendications

1. **Évacuateur de vessie** (10)(*figure 1*) pour
- alternativement introduire et retirer une fluide (50) dans une
- cavité de vessie
- au travers d'une conduite,
comprenant
- un **récipient** (12) pour le **fluide**
- définissant
- un volume récepteur de **fluide** et
- ayant
- une **ouverture de col** (32),
- des **moyens de filtrage**
- disposés à l'intérieur du volume de ce récipient (12)
- pour la séparation de particules tissulaires (52) de dimension supérieure à une valeur prédéterminée
- d'avec le fluide introduit depuis ledit volume au travers de l'ouverture de col (32) et
- des **moyens formant bouchon** (16)
- pour interconnecter l'ouverture de col (32) et la conduite,
- un **élément filtrant** allongé (14) des **moyens de filtrage**
- étant disposé au voisinage de l'ouverture de col (32) et
- s'étendant à l'intérieur du volume du récipient (12) et
- étant disposé autour de l'**axe** (F) de l'ouverture de col et
- définissant
- deux **ouvertures axiales** (24,26)
- une **supérieure** (24), à l'ouverture de col (32) et
- une **inférieure** (26), à distance de la précédente
- de manière à définir un passage axial entre ces deux ouvertures,
- ce passage fonctionnant à la manière d'une dérivation,
- de manière que les matières s'écoulant au travers de ce passage
- ne traversent pas l'élément filtrant (14);
- cet **évacuateur de vessie** (10) étant conçu
- pour l'**application** de **forces de compression** (F_{c}) (*figure 2*) sur le récipient (12)
- dans des directions **transversales** audit axe (F) de l'élément filtrant (14)
- pour solliciter le fluide (G) se trouvant à l'intérieur du volume du récipient (12)
- essentiellement dépourvu de particules tissulaires (52) de dimensions dépassant ladite valeur prédéterminée,
- à traverser l'ouverture de col (32) jusque dans la conduite en direction de la cavité de vessie, et
- au **relâchement** des **forces de compression** (F_{c}) (*figure 3*),
- le récipient (12) étant conçu
- de manière à se dilater
- afin d'extraire le fluide portant les particules tissulaires non filtrées (H) vers le volume du récipient (12)
- via l'ouverture de col (32),
***caractérisé en*** ce que
- le récipient (12) est conçu
- de manière à être comprimé par les forces de compression (F_{c})
- en une **partie supérieure** (18) de lui-même, tandis que
- la **longueur** de l'élément filtrant (14)
- s'étendant dans le volume du récipient (12) est telle que
- l'**ouverture** axiale **inférieure** (26) de l'élément filtrant (14) soit
- placée **à distance** (c'est-à-dire en une position inférieure)
- par rapport à
- la **partie supérieure** (18) du récipient (12)
- où sont appliquées les **forces de compression** (Fc),
- cette configuration faisant en sorte que
- le **fluide** (G) (figure 2)
- sollicité par les forces de compression (F_{c})
- soit dirigé contre la surface extérieure de la paroi latérale de l'élément filtrant (14),
- avec le fluide (G) s'écoulant au travers de ses **pores de filtrage** (36),
- tandis que, au relâchement des forces de compression (F_{c}) (*figure* 3)
- le fluide emportant les particules tissulaires **non filtrées** (H) s'écoule
- généralement **axialement** à l'intérieur de l**'élément filtrant** (14)
- au travers de son ouverture axiale **inférieure** (26)
- en passant par ses **pores de filtrage** (36).

2. Evacuateur de vessie selon la revendication 1,
*caractérisé en* ce que
- le **récipient** (12) comprend
- une partie de corps généralement cylindrique.

3. Evacuateur de vessie selon la revendication 1,
*caractérisé en* ce que
- **l'élément filtrant** (14) est
- de forme générale cylindrique.

4. Evacuateur de vessie selon la revendication 1,
*caractérisé en* ce que
- les **parois latérales** du récipient (12) et de l'élément filtrant (14) sont
- essentiellement parallèles.

5. Evacuateur de vessie selon la revendication 1,
*caractérisé en* ce que
- le **récipient** est constitué d'un
- **matériau élastique**.

6. Evacuateur de vessie selon la revendication 5,
*caractérisé en* ce que
- le **matériau élastique** est
- un matériau **polymère**
- choisi dans le groupe comprenant
- le polychlorure de vinyle (PVC), le polyéthylène et le polypropylène.

7. Evacuateur de vessie selon la revendication 1,
*caractérisé en* ce que
- les **moyens formant bouchon** (16) comprennent
- une **première partie** (40)
- s'étendant depuis le **col ouvert** (32) et
- une **seconde partie** (42)
- plus proche de ladite **conduite**,
- les **axes** de cette première partie (40) et de cette seconde partie (42) définissant
- un **angle aigu** (D).

8. Evacuateur de vessie selon la revendication 7,
*caractérisé en* ce que
- ledit **angle** (D) est de l'ordre de
- **45**° environ.

9. Evacuateur de vessie selon la revendication 1,
*caractérisé en* ce que
- **l'élément filtrant** (14) comprend
- un **tamis** (34)
- possédant
- lesdites **ouvertures des pores de filtrage** (36)
- de l'ordre d'environ **1 mm** ou moins.

10. Evacuateur de vessie selon la revendication 9,
*caractérisé en* ce que
- les **ouvertures des pores de filtrage** (36) sont
- d'environ **0,5** à **1,0 mm**.

11. Evacuateur de vessie selon la revendication 1,
*caractérisé en* ce que
- ladite **conduite** comprend
- un canal opératoire d'un endoscope.
